# EUROPEAN PATENT APPLICATION

(11) **EP 2 500 034 A1**
(43) Date of publication of application: **19.09.2012**
(21) Application number: 10830062.5
(22) Date of filing: 12.11.2010
(51) Int. Cl.: A61K 39/395, A61P 17/00, A61P 17/04, A61P 37/08

(54) **THERAPEUTIC AGENT FOR PSORIASIS OR ATOPIC DERMATITIS**

(30) Priority: 13.11.2009 JP 2009260031
(71) Applicant: Kabushiki Kaisha Saiwai Medix, Tokyo 101-0024 (JP)
(72) Inventor: SHICHIRI, Masayoshi, Tokyo 174-0065 (JP)
(74) Representative: Denison, Christopher Marcus
(86) International application number: PCT/JP2010/070627
(87) International publication number: WO 2011/059110

(57) **Abstract**

The present invention provides a therapeutic agent for psoriasis or atopic dermatitis, which comprises *anti-Staphylococcus-aureus* antibodies as an active ingredient. Specifically, the following is provided: a therapeutic agent for psoriasis or atopic dermatitis, which comprises, as an active ingredient, comprehensive *anti-Staphylococcus-aureus* surface antibodies comprising antibodies raised against entire protein molecules expressed on the surface of *Staphylococcus aureus,* which is produced by: (a) treating *Staphylococcus aureus* with a protein crosslinking/fixation reagent to fix proteins expressed on the surface of *Staphylococcus aureus* via intramolecular crosslinking; (b) administering, as an immunogen, *Staphylococcus aureus* treated with the protein crosslinking/fixation reagent for protein fixation to an animal; and (c) obtaining an antibody from the animal.

## Description

### Technical Field

The present invention relates to a therapeutic agent for psoriasis or atopic dermatitis that comprises an antibody as an active ingredient.

### Background Art

The pathogenesis of psoriasis and of atopic dermatitis still remains to be elucidated, but they are categorized as allergic skin diseases. Although a complex and complicated interaction among a variety of genetic/environmental factors has been known to play a central role for the development of atopic dermatitis, exact pathogenetic mechanisms remain largely unknown (Non-Patent Literature 1). Numerous factors have been studied thus far in search for possible causes/triggers involved in the development or progression of the diseases. These include bacteria, such as *Staphylococcus aureus,* a resident bacterium on the human skin. However, it has been concluded that such bacteria do not cause or aggravate atopic dermatitis. The pathogenetic mechanism of psoriasis is less well elucidated, rendering its proper treatment unfeasible (Non-Patent Literature 2). As a result, at present, adrenocortical steroid hormones that act against allergic mechanisms are often used as their remedies, despite their unwanted action to promote bacterial proliferation.

*Staphylococcus aureus* is a facultative anaerobic gram-positive coccus. It has been reported that *Staphylococcus aureus* is a resident bacterium present on the skin, pores, or the nasal cavity of a human and enters a human body through wounds and the like so as to cause a variety of suppurative diseases, pneumonia, sepsis, meningitis, and the like. Also, it has been reported that *Staphylococcus aureus* toxin causes food poisoning and toxic shock syndrome.

A variety of proteins are expressed in microorganisms such as bacterial cells and viruses. Hitherto, there have been attempts to select particular antigen protein molecules that are thought to be useful from among such proteins, to produce mainly monoclonal antibodies, and to use the antibodies for medical care and the like. However, there are limitations to the use of an antibody that recognizes only a single antigen molecule (expressed on the surfaces of bacterial cells or the like) for the purpose of recognizing a specific cell and targeting the cell.

In general, antibiotics have been mainly used to prevent or treat diseases such as infectious diseases caused by bacteria, viruses, and the like. In addition, it has been common to administer a vaccine that has been produced using an attenuated virus to a living body so as to produce an antiviral antibody therein for prevention or treatment of a viral infection. However, when an antibiotic is used, it is necessary to select a proper antibiotic depending on the type of bacterium. In addition, the use of antibiotics results in generation of drug-resistant strains, which has been problematic. Further, when a vaccine is used, the safety of the vaccine must to be considered. Therefore, infectious diseases for which vaccines can be used are limited.

### Citation List

### Non-Patent Literature

Non-Patent Literature 1: Bieber T, Novak N: Pathogenesis of atopic dermatitis: new developments, Current Allergy and Asthma Reports, 2009, 9 291-294
Non-Patent Literature 2: Wippel-Slupetzky, K. Stingl, G: Future perspectives in the treatment of psoriasis, Current Problems in Dermatology, 2009, 38:172-89

### Summary of the Invention

The present invention is to provide a therapeutic agent for psoriasis or atopic dermatitis, which comprises, as an active ingredient, *anti-Staphylococcus-aureus* antibodies, and in particular, comprehensive (wide-variety of) antibodies raised against entire proteins expressed on the surface of *Staphylococcus aureus.*

The present inventor assumed that psoriasis and atopic dermatitis represent skin lesions caused by a skin resident bacterium, *Staphylococcus aureus,* and/or those affected by toxins produced by *Staphylococcus aureus* at erosive scratched skin sites. The present inventors raised *anti-Staphylococcus-aureus* antibodies by the method for producing comprehensive anti-surface antigen antibodies using, as an antigen, a microorganism treated with a protein crosslinking/fixation reagent for protein fixation (JP Patent Application No. 2008-324257). Then, the present inventors conducted intensive studies to elucidate whether or not the *anti-Staphylococcus-aureus* antibodies can be used for treatment of psoriasis and atopic dermatitis.

First, the present inventor hypothesized that a toxin produced by *Staphylococcus aureus* could be an aggravating factor of psoriasis and atopic dermatitis, based on the recognition that a toxin produced by *Staphylococcus aureus* is a superantigen that activates T cells in peripheral blood.

The present inventor directly used, as antigens, *Staphylococcus aureus* cells that had been surface-treated with formaldehyde for protein fixation to immunize animals and produced comprehensive polyclonal antibodies against entire antigen protein molecules that had been fixed on the surface of *Staphylococcus aureus.*

Repeatedly application of the *anti-Staphylococcus-aureus* surface antibodies to the lesions resulted in complete elimination of *Staphylococcus aureus* from the lesions, enabling to treat psoriasis and atopic dermatitis. This led to the completion of the present invention.

Specifically, the present invention is described below.
[1] A therapeutic agent for psoriasis or atopic dermatitis, which comprises *anti-Staphylococcus-aureus* antibodies as an active ingredient.
[2] The therapeutic agent for psoriasis or atopic dermatitis according to [1], wherein the *anti-Staphylococcus-aureus* antibodies are comprehensive *anti-Staphylococcus-aureus* surface antibodies comprising antibodies raised against entire protein molecules expressed on the surface of *Staphylococcus aureus,* which is produced by: (a) treating *Staphylococcus aureus* with a protein crosslinking/fixation reagent to fix proteins expressed on the surface of *Staphylococcus aureus* via intramolecular crosslinking; (b) administering, as an immunogen, *Staphylococcus aureus* treated with the protein crosslinking/fixation reagent for protein fixation to an animal; and (c) obtaining an antibody from the animal.
[3] The therapeutic agent for psoriasis or atopic dermatitis according to [2], wherein the protein crosslinking/fixation reagent is selected from the group consisting of formaldehyde, paraformaldehyde, and glutaraldehyde.
[4] The therapeutic agent for psoriasis or atopic dermatitis according to [2], wherein the protein crosslinking/fixation reagent is 1% to 38% (v/v) formalin.
[5] The therapeutic agent for psoriasis or atopic dermatitis according to any one of [2] to [4], wherein protein fixation is carried out using a protein crosslinking/fixation reagent for 10 minutes to 48 hours.
[6] The therapeutic agent for psoriasis or atopic dermatitis according to any one of [2] to [5], wherein an animal to which an immunogen is administered is a chicken.
[7] The therapeutic agent for psoriasis or atopic dermatitis according to [6], wherein antibodies are obtained from an egg laid by an immunogen-administered treated chicken.

This description includes part or all of the contents as disclosed in the description and/or drawings of Japanese Patent Application No. 2009-260031, which is a priority document of the present application.

### Brief Description of Drawings

Fig. 1 shows photos indicating effects of the therapeutic agent of the present invention upon atopic dermatitis patients (I).
Fig. 2 shows photos indicating effects of the therapeutic agent of the present invention upon atopic dermatitis patients (II).
Fig. 3 shows photos indicating effects of the therapeutic agent of the present invention upon atopic-dermatitis-affected dogs (I).
Fig. 4 shows photos indicating effects of the therapeutic agent of the present invention upon atopic-dermatitis-affected dogs (II).
Fig. 5 shows photos indicating effects of the therapeutic agent of the present invention upon atopic-dermatitis-affected dogs (III).
Fig. 6 shows photos indicating effects of the therapeutic agent of the present invention upon psoriasis patients.

### Description of Embodiments

The present invention is described below in detail.

The therapeutic agent for psoriasis or atopic dermatitis of the present invention comprises *anti-Staphylococcus-aureus* antibodies as an active ingredient. The *anti-Staphylococcus-aureus* antibodies can be produced using the whole or a part of *Staphylococcus aureus* via a conventionally known method. The *anti-Staphylococcus-aureus* antibodies of the present invention may be polyclonal antibodies or monoclonal antibodies. The *anti-Staphylococcus-aureus* antibodies include not only complete antibodies but also functional fragments of antibodies. The term "functional fragment of an antibody" refers to a part of an antibody (i.e., partial fragment) maintaining at least one action of the antibody against the corresponding antigen. Specific examples of functional fragments include F(ab')₂, Fab', Fab, Fv, disulfide bond Fv, single chain Fv (scFv), and polymers thereof (D. J. King., Applications and Engineering of Monoclonal Antibodies, 1998, T. J. International Ltd.).

In addition, monoclonal antibodies used herein may be of one type. Alternatively, two or more types of monoclonal antibodies that recognize different epitopes (e.g., 2, 3, 4, or 5 types of monoclonal antibodies) may be used. Further, the *anti-Staphylococcus-aureus* antibodies of the present invention also include recombinant antibodies that have been artificially modified to reduce heterologous antigenicity to humans. Examples of such antibodies include a chimeric antibody, a humanized antibody, and a human antibody, each of which can be produced by a conventionally known method.

Preferably, the above antibodies is polyclonal antibodies such as comprehensive surface antibodies described below.

For production of comprehensive surface antibodies, the surfaces of *Staphylococcus aureus* cells are treated with formaldehyde for protein fixation, and the treated cells are directly used as antigens. By doing so, comprehensive antibodies against *Staphylococcus aureus* can be produced. Here, the production of comprehensive antibodies against *Staphylococcus aureus* refers to the production of antibodies against entire molecules of the many antigen protein molecules expressed on the surface of *Staphylococcus aureus* comprehensively at once. The thus produced antibody is termed herein as comprehensive (wide-variety of) *anti-Staphylococcus-aureus* surface antibodies. Comprehensive *anti-Staphylococcus-aureus* surface antibodies are polyclonal antibody comprising individual antibodies against many antigen proteins expressed on the surface of *Staphylococcus aureus.* According to the method of the present invention, comprehensive *anti-Staphylococcus-aureus* surface antibodies comprising antibodies against enitre surface protein molecules can be obtained by simply administering *Staphylococcus aureus* treated for protein fixation to animals. Here, antibodies against enitre surface protein molecules do not necessarily comprise antibodies against all proteins expressed on the surface of *Staphylococcus aureus.* However, they desirably comprise antibodies against almost all surface proteins that are highly abundant.

The comprehensive *anti-Staphylococcus-aureus* antibodies can be produced by the method described below.

First, *Staphylococcus aureus* is treated with an intramolecular protein crosslinking/fixation reagent for protein fixation. Examples of an intramolecular protein crosslinking/fixation reagent include aldehydes such as formaldehyde, paraformaldehyde, and glutaraldehyde. Preferably, fixation is carried out using formalin, which is a 35% to 38% (and preferably 37%) aqueous formaldehyde solution.

If such intramolecular protein crosslinking/fixation reagent is used as a fixation reagent, the reagent permeates *Staphylococcus aureus* cells and then aldehyde in the reagent binds to an amino group (α-amino or ε-amino of a lysine residue) or an SH group of a protein present on the surface of each *Staphylococcus aureus* cell so as to form an intermolecular crosslink. This results in protein coagulation/denaturation. Such crosslinking causes destruction of protein conformation. Accordingly, various bioactivities related to enzyme activity, transport, secretion, and the like are inhibited. The intramolecular protein crosslinking/fixation reagent acts to induce a protein molecule crosslinking reaction. Thus, such fixation does not influence other substances such as fat, resulting in leakage of fat and the like.

If a commercially available 35% to 38% aqueous formaldehyde solution (i.e., formalin) is used, fixation can be carried out using 1% to 38% (v/v) formalin, preferably 2% to 20% (v/v) formalin, and further preferably 5% to 10% (v/v) formalin. Here, formalin to be used may be prepared using distilled water, physiological saline, buffer, or the like.

Then, *Staphylococcus aureus* is homogenized. Formalin (5 to 20 ml) is added to pellets of the homogenized product (1 g). For fixation, formalin is added to the homogenized product of *Staphylococcus aureus.* The mixture is stirred and then allowed to stand still at 4°C to 30°C for 30 minutes to 48 hours or longer. Viable *Staphylococcus aureus* cells are deactivated via short-time formalin treatment. However, according to the method of the present invention, deactivation of *Staphylococcus aureus* alone is insufficient, and thus it is necessary to carry out formalin treatment to an extent that causes intramolecular crosslinking of protein molecules expressed on the surface of *Staphylococcus aureus.*

The homogenized product of *Staphylococcus aureus* treated for fixation is diluted with physiological saline or buffer (according to need), suspended, and mixed with a known adjuvant. The resultant can be administered as an immunogen to animals. Examples of animals that can be used include mammals (such as mice, rats, nutrias, rabbits, sheep, goats, horses, and cattle) and birds (such as chickens and ostriches). Of these, birds such as chickens are preferable because chicken eggs containing IgY antibodies can be obtained.

Animals can be immunized using the immunogen via a conventional method. For instance, such method is generally carried out via intraperitoneal or subcutaneous injection of the immunogen to animals. Immunization may be performed once. However, it is preferable to administer the immunogen several times every 2 to 10 days. In addition, a booster may be given every 5 to 10 days, several times in total after the initial administration.

A method for preparing a *Staphylococcus aureus* immunogen is described below. The method described below is an example, and thus the present invention is not limited thereto.

*Staphylococcus aureus* cells are collected by centrifugation and formed into pellets. The pellets are immersed and dispersed via stirring in formalin. The resultant is left for 30 minutes and filtered through coarsely meshed filter paper to remove residues. The obtained filtrate is collected and centrifuged to prepare pellets. Phosphate buffer is added to the pellets. The resultant is used as an immunogen.

The comprehensive anti-surface antibody composition of the present invention can be obtained from serum of an animal immunized with the above immunogen. In addition, if an animal to be immunized is a chicken, the antibodies can be obtained from chicken eggs laid by the chicken. Chicken eggs containing the antibodies with a high antibody titer can be obtained about 3 months after final immunization. Chicken egg yolk contains IgY and chicken egg albumen contains IgA and IgM. If the antibodies are collected from whole eggs, the comprehensive antibody composition contains IgY, IgA, and IgM. If the antibodies are collected from egg yolk, the composition contains IgY. When the antibody composition of the present invention are prepared using chicken eggs, whole eggs or egg yolk alone can be used.

The obtained chicken eggs are broken. Egg yolk and albumen are stirred together or egg yolk alone is stirred, followed by powderization. Powderization is preferably carried out at 60°C or below. This is because antibodies, which are proteins, are weak against heat, and thus heating at 70°C or above causes a loss of antibody activity. Preferably, drying is carried out via lyophilization, spray drying, or hot-air drying at 60°C or below for powderization. If necessary, powderized chicken eggs are introduced into a pulverizer to obtain a fine powder. A powder obtained using the above method contains egg yolk oil components, and thus has moisture. The oil components may be removed therefrom by complete defatting via an ultracold critical method.

Herein, the comprehensive anti-surface antibody composition obtained from chicken eggs is sometimes referred to as a "chicken egg antibodies."

The antibody titer of the obtained antibodies can be determined by ELISA (enzyme-linked immunosorbent assay), EIA (enzyme immunoassay), RIA (radioimmunoassay), a fluorescent antibody method, or the like. The antibody titer may be determined using serum obtained from a subject animal. In addition, if chicken eggs are used, the antibody titer of antibodies in an extract from chicken eggs may be determined.

The comprehensive antibodies against *Staphylococcus aureus* of the present invention can be used for prevention or treatment of psoriasis and atopic dermatitis. The comprehensive *anti-Staphylococcus-aureus* antibodies of the present invention contain antibodies against entire protein molecules expressed on the surface of *Staphylococcus aureus.* Therefore, they are highly destructive to *Staphylococcus aureus* and thus they can kill, eliminate, or remove *Staphylococcus aureus.*

For example, the prophylactic or therapeutic agent for psoriasis and atopic dermatitis, which comprises the comprehensive *anti-Staphylococcus-aureus* antibodies as an active ingredient, can be administered to animals. The antibody composition can be administered via the oral route, the nasal route, the parenteral route such as via intravenous, intramuscular, subcutaneous, or intraperitoneal injection, or the non-enteral route. In particular, they are formed into liniment such as ointment and thus can be locally administered to skin lesions via application or liquid spraying. Such ointment contains, as a carrier, fat, fatty oil, lanolin, vaseline, paraffin, Plastibase®, wax, plaster, resin, plastic, glycols, higher alcohol, glycerin, water or an emulsifier, and/or a suspending agent. The dose of the pharmaceutical composition of the present invention to be administered varies depending on symptoms, age, weight, and the like. However, in general, it can be orally administered to an adult at about 0.01 mg to 10000 mg per day in the form of single dose or multiple doses.

The prophylactic or therapeutic antibody composition for psoriasis or atopic dermatitis may comprise a carrier, diluent, or excipient generally used in the field of drug formulation. Examples of a carrier or excipient for tablets include lactose and magnesium stearate.

The therapeutic agent for psoriasis or atopic dermatitis of the present invention can be used for animals at risk of contracting psoriasis or atopic dermatitis. Examples of such animals include humans, monkeys, and pet animals such as dogs and cats.

The use of the therapeutic agent comprising *anti-Staphylococcus-aureus* antibodies of the present invention enables elimination of *Staphylococcus aureus* from lesions of psoriasis and atopic dermatitis, making it possible to prevent aggravation of skin damage such as erosion of scratch wounds on skin surfaces due to *Staphylococcus aureus* toxin.

The present invention is hereafter described in greater detail with reference to the following examples, although the technical scope of the present invention is not limited thereto.

### Example 1: Production of comprehensive antibodies against an antigen (Staphylococcus aureus)

### (1) Antigen preparation

A *Staphylococcus aureus* strain was cultured to prepare frozen *Staphylococcus aureus* pellets. The *Staphylococcus aureus* strain used herein was a strain provided by MicroBiologics (purchased from Kanto Chemical Co., Ltd.).

*Staphylococcus aureus* pellets (about 3 mg) were thawed and introduced into a 15-ml capped test tube. A 5% formalin solution (5 ml) was added thereto. The cap was hermetically sealed.

Mixing was carried out several times using a test tube mixer (for approximately 10 minutes in total) so as to cause uniform diffusion and dissolution. Thus, the pellets were completely dissolved in the 5% formalin solution for fixation of *Staphylococcus aureus* surface antigens, followed by incubation. After incubation, the resultant was left for 30 minutes.

The 5% formalin solution in which the pellets had been dissolved was introduced into a centrifuge. The bottom-phase solution (1.5 ml) containing the sediment of the centrifuged solution was suctioned using a pipette.

Natural filtration or suction filtration was conducted using coarsely meshed filter paper or a filter, followed by centrifugation. The supernatant was discarded to reduce the formalin concentration.

The sediment was dissolved in physiological saline. The resultant was used as an antigen for administration.

The antigen (1 ml) and white oil were mixed in a stepwise manner using an emulsion pump to prepare an emulsion (emulsified liquid). The obtained emulsion was administered to animals.

### (2) Immunization

The emulsion (0.5 ml) prepared in (1) was administered to inguinal regions of 5-week-old male chickens. Upon administration, the antigen composition for administration was heated to the chicken body temperature (37°C) in order to reduce invasiveness to the chickens.

The same dose of the emulsion was given as a booster to the chickens every 7 days, twice in total after the initial administration.

Whole eggs laid by the immunized chickens were broken and stirred. A dried egg powder was produced using a GA22 spray dryer (Yamato Scientific Co., Ltd.), a hot air dryer, and a pulverizer. The spray dryer temperature was adjusted to 60°C or below.

ELISA was conducted for antibody titer determination.

### Example 2: Treatment of human atopic dermatitis

The antibody titer threshold was determined for the chicken egg antibody produced by the method described in Example 1. The polyclonal antibody was mixed with petrolatum ointment to prepare an ointment formulation for treatment in a manner such that the antibody accounted for 1% to 5% of the formulation weight. The prepared ointment formulations were applied to lesions of atopic dermatitis patients.

The threshold determination results showed that strong itching sensation and redness induced by *Staphylococcus aureus* toxin (SA toxin) were reduced at each concentration.

The polyclonal antibody was mixed with petrolatum ointment in a manner such that the antibody accounted for 5% of the formulation weight for further examination.

The ointment formulation was applied to 10 atopic dermatitis patients. The ointment formulation was applied once or twice daily. The effects of the ointment formulation were exhibited within about 2 weeks, and remarkable effects were observed within 2 to 3 months.

Table 1 shows results of the ointment effect evaluation test. As shown in table 1, 90% of the cases showed "Improved" or better results. Thus, the efficacy rate was found to be very high.

**Table 1**

| Ointment effect evaluation test results | | | | |
|---|---|---|---|---|
| Subject | Sex | Age | Administration period | Effect evaluation |
| A | ♂ | 35 | 6 months (completed) | (III) Very effective: Photos attached |
| B | ♂ | 28 | 6 months (continued) | (I) Relatively improved: Disappearance of the itching sensation, attenuation of redness |
| C | ♂ | 32 | 3 months (completed) | (III) Very effective: Recovery of skin |
| D | ♀ | 24 | 6 months (completed) | (III) Very effective: Recovery of skin |
| E | ♂ | 8 | 1 month (completed) | (-) Not effective: No improvement of symptoms |
| F | ♀ | 21 | 2 months (continued) | (II) Effective: Disappearance of itching sensation and redness |
| G | ♂ | 40 | 8 months (continued) | (I) Relatively improved: Poor compliance |
| H | ♀ | 34 | 3 months (completed) | (III) Very effective: Recovery of skin |
| I | ♀ | 24 | 6 months (completed) | (III) Very effective: Recovery of skin |
| J | ♀ | 31 | 4 months (completed) | (III) Very effective: Recovery of skin |

| | | | | |
|---|---|---|---|---|
| Effect evaluation criteria (III) Very effective: Disappearance of itching sensation and redness and recovery to normal skin (II) Effective: Disappearance of itching sensation and redness (I) Relatively improved: Disappearance of itching sensation or redness (-) Not effective: No exhibited effects | | | | |

Cases with "Relatively improved" or better results accounted for 90% (efficacy rate). Among them, cases with "Very effective" results accounted for 60%, cases with "Effective" results accounted for 10%, and cases with "Relatively improved" results accounted for 20%, while cases with "Not effective" results accounted for 10%.

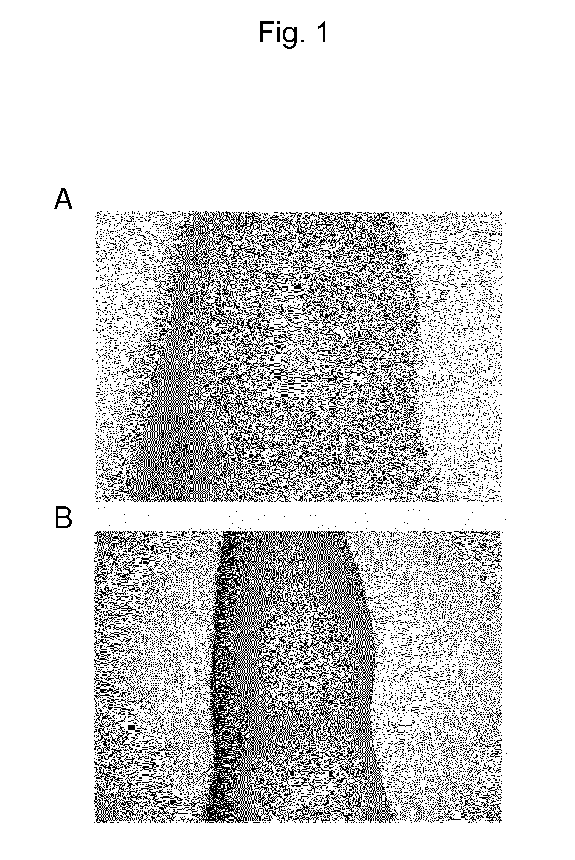

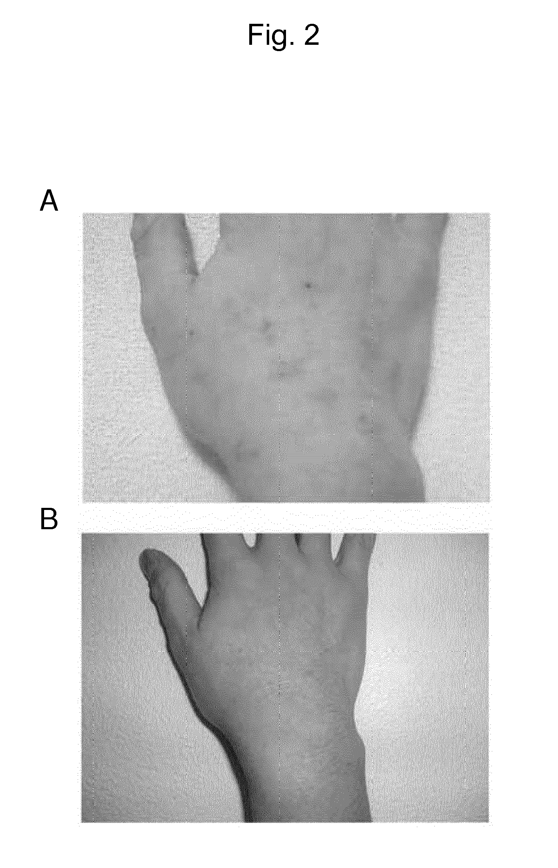

In addition, figs. 1 and 2 show photos indicating the effects of the therapeutic agent of the present invention upon atopic dermatitis patients. Figs. 1A and 2A show lesions of atopic dermatitis patients before treatment with the use of the therapeutic agent of the present invention. Figs. 1B and 2B show lesions of atopic dermatitis patients after treatment with the use of the therapeutic agent of the present invention. As shown in the figures, it is understood that the use of the therapeutic agent of the present invention resulted in disappearance or alleviation of symptoms observed in lesions of atopic dermatitis patients.

### Example 3: Treatment of atopic dermatitis of dogs

Dogs have a life expectancy of about 10 to 20 years. Dogs aged 5 and older are highly likely to develop atopic dermatitis. In this Example, effects of the therapeutic agent of the present invention upon atopic dermatitis of dogs were evaluated.

Ointment was prepared by the method described in Example 2 using the *anti-Staphylococcus-aureus* antibody produced by the method described in Example 1. The ointment was applied to lesions of 7 dogs with atopic dermatitis once daily during a series of consecutive days.

Table 2 shows the effect evaluation test results. As shown in table 2, improvement of atopic dermatitis was confirmed for many cases.

**Table 2**

| Test for verifying effects of SA-antibody-containing ointment upon dog atopic dermatitis | | | | |
|---|---|---|---|---|
| Case | Dog breed / Sex | Age | Administration period | Effect evaluation |
| Case 1 | Long dachs / ♂ | 7 | 1 month (completed) | (III) Very effective: Recovery of skin |
| Case 2 | Miniature dachs / ♀ | 9 | 1.5 months (completed) | (III) Very effective: Recovery of skin |
| Case 3 | Miniature dachs / ♂ | 15 | 1.5 months (continued) | (II) Effective: Disappearance of itching sensation and redness |
| Case 4 | Shih Tzu / ♀ | 12 | 1.5 months (continued) | (-) No improvement of symptoms |
| Case 5 | Pug / ♀ | 11 | 1 month (continued) | (-) No improvement of symptoms |
| Case 6 | Shiba inu / ♀ | 10 | 1 month (continued) | (-) No improvement of symptoms |
| Case 7 | Papillon / ♂ | 2 | 1 month (continued) | (II) Effective: Disappearance of itching sensation and redness |

| | | | | |
|---|---|---|---|---|
| Effect evaluation criteria (III) Very effective: Disappearance of itching sensation or redness and recovery to normal skin (II) Effective: Disappearance of redness (I) Relatively improved: Disappearance of itching sensation and redness (-) No improvement of symptoms | | | | |

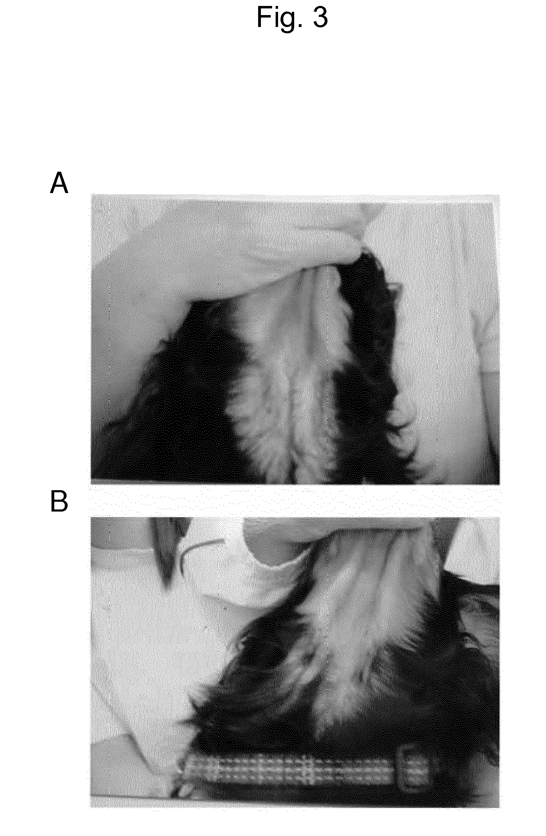

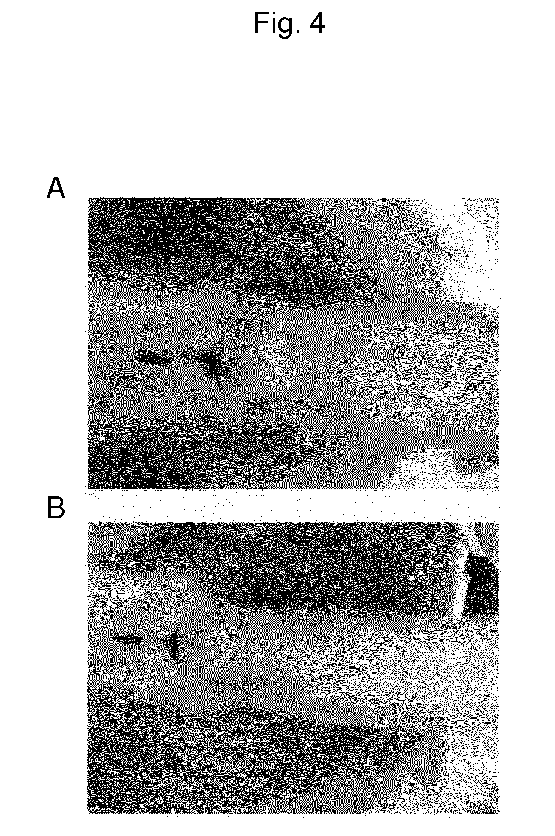

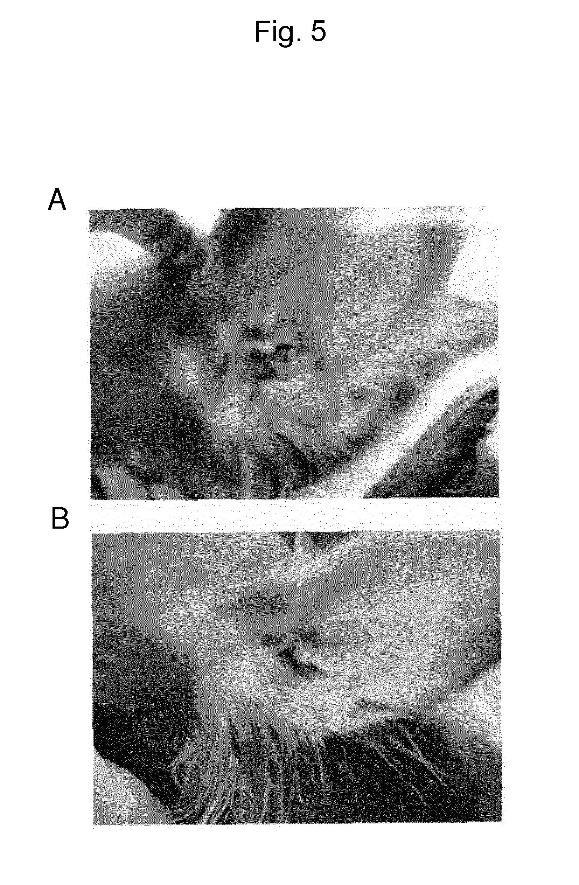

Cases with "Relatively improved" or better results accounted for 57% (efficacy rate). Among them, cases with "Very effective" results accounted for 29%, cases with "Effective" results accounted for 29%, and cases with "Relatively improved" results accounted for 0%, while cases with "No improvement" results accounted for 42%.
In addition, figs. 3 and 5 show photos indicating the effects of the therapeutic agent of the present invention upon dogs affected with atopic dermatitis. Figs. 3A, 4A, and 5A show lesions of dogs affected with atopic dermatitis before treatment with the use of the therapeutic agent of the present invention. Figs. 3B, 4B, and 5B show lesions of dogs affected with atopic dermatitis after treatment with the use of the therapeutic agent of the present invention. As shown in the figures, it is understood that the use of the therapeutic agent of the present invention resulted in disappearance or alleviation of symptoms observed in lesions of dogs affected with atopic dermatitis. In this Example, it was found that the effects confirmed for dogs were relatively weaker than those for humans in Example 2. The onset of effects was delayed for a longer period of time in older dogs, and in particular, dogs aged 10 and older.

### Example 4: Treatment of human psoriasis

Ointment was prepared by the method described in Example 2 using the *anti-Staphylococcus-aureus* antibody produced according to the method described in Example 1. The ointment was applied once or twice daily to lesions of psoriasis patients. The effects of the ointment were confirmed within about 2 weeks and significant effects were exhibited within 2 to 3 months. The lesions were observed for a certain period of time after the start of application.

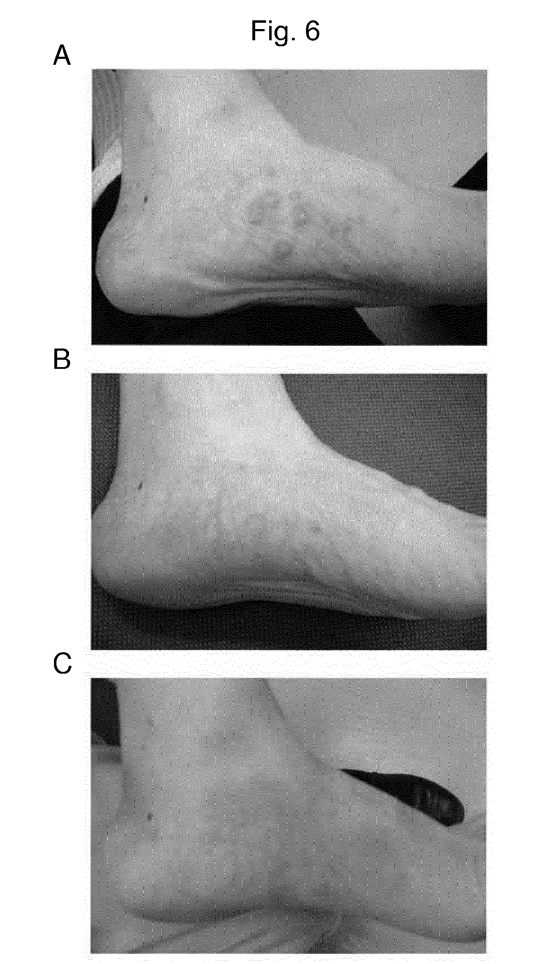

Fig. 6 shows photos indicating effects of the therapeutic agent of the present invention upon psoriasis patients. Figs. 6A, 6B, and 6C show photos indicating conditions of lesions, which were taken on days 15, 30, and 90, respectively, after application. As shown in the figures, continuous application resulted in alleviation of symptoms.

### Industrial Applicability

Hitherto, it had been believed that no direct causal relationship existed between *Staphylococcus aureus* and psoriasis/atopic dermatitis. The therapeutic agent for psoriasis or atopic dermatitis of the present invention comprises, as an active ingredient, *anti-Staphylococcus-aureus* antibodies and causes elimination of *Staphylococcus aureus* from skin lesions. Thus, it can be used for treatment of psoriasis or atopic dermatitis. In particular, the comprehensive *anti-Staphylococcus-aureus* antibodies of the present invention comprises antibodies against entire proteins expressed on the surface of *Staphylococcus aureus,* which are produced by administering proteins (expressed on the surface of *Staphylococcus aureus* and fixed thereon via intramolecular crosslinking with the use of an intramolecular protein crosslinking reagent) to animals. An antibody composition comprising such comprehensive antibodies is imparted with high levels of specificity and sensitivity to *Staphylococcus aureus* that have been impossible to impart by conventional antibody production methods. A pharmaceutical composition comprising the antibodies can be effectively used for treatment of psoriasis and atopic dermatitis.

The composition comprising the *anti-Staphylococcus-aureus* antibodies of the present invention as an active ingredient can be used for treatment of psoriasis or atopic dermatitis.

All publications, patents, and patent applications cited herein are incorporated herein by reference in their entirety.

## Claims

1. A therapeutic agent for psoriasis or atopic dermatitis, which comprises an *anti-Staphylococcus-aureus* antibodies as an active ingredient.

2. The therapeutic agent for psoriasis or atopic dermatitis according to claim 1, wherein the *anti-Staphylococcus-aureus* antibodies are comprehensive *anti-Staphylococcus-aureus* surface antibodies comprising antibodies raised against entire protein molecules expressed on the surface of *Staphylococcus aureus,* which is produced by: (a) treating *Staphylococcus aureus* with a protein crosslinking/fixation reagent to fix proteins expressed on the surface of *Staphylococcus aureus* via intramolecular crosslinking; (b) administering, as an immunogen, *Staphylococcus aureus* treated with the protein crosslinking/fixation reagent for protein fixation to an animal; and (c) obtaining an antibody from the animal.

3. The therapeutic agent for psoriasis or atopic dermatitis according to claim 2, wherein the protein crosslinking/fixation reagent is selected from the group consisting of formaldehyde, paraformaldehyde, and glutaraldehyde.

4. The therapeutic agent for psoriasis or atopic dermatitis according to claim 2, wherein the protein crosslinking/fixation reagent is 1% to 38% (v/v) formalin.

5. The therapeutic agent for psoriasis or atopic dermatitis according to any one of claims 2 to 4, wherein protein fixation is carried out using a protein crosslinking/fixation reagent for 10 minutes to 48 hours.

6. The therapeutic agent for psoriasis or atopic dermatitis according to any one of claims 2 to 5, wherein an animal to which an immunogen is administered a chicken.

7. The therapeutic agent for psoriasis or atopic dermatitis according to claim 6, wherein antibodies are obtained from an egg laid by an immunogen-administered chicken.
